# EUROPEAN PATENT SPECIFICATION

(11) **EP 3 641 653 B1**
(45) Date of publication and mention of the grant of the patent: **16.03.2022**
(21) Application number: 18824141.8
(22) Date of filing: 19.06.2018
(51) Int. Cl.: A61B 6/03, G06T 15/10, A61C 19/045, A61C 11/00, G16H 30/40, G16H 50/50

(54) **METHOD OF RECORDING OF TEMPOROMANDIBULAR JOINT MOVEMENT AND GEOMETRY**
VERFAHREN ZUM AUFZEICHNEN EINER KIEFERGELENKBEWEGUNG UND -GEOMETRIE
PROCÉDÉ D'ENREGISTREMENT DU MOUVEMENT ET DE LA GÉOMÉTRIE DE L'ARTICULATION TEMPORO-MANDIBULAIRE

(30) Priority: 24.06.2017 PL 42201517
(43) Date of publication of application: 29.04.2020
(73) Proprietor: Walerzak, Konrad, 02-434 Warszawa (PL)
(72) Inventor: WALERZAK, Sebastian, 02-419 Warszawa (PL); WALERZAK, Monika, 02-419 Warszawa (PL); SYBILSKI, Kamil, 05-820 Piastow (PL); MALACHOWSKI, Jerzy, 05-120 Legionowo (PL); DAMAZIAK, Krzysztof, 01-707 Warszawa (PL); WALERZAK, Konrad, 02-434 Warszawa (PL)
(74) Representative: Pawlowska, Justyna
(86) International application number: PCT/PL2018/050027
(87) International publication number: WO 2019/004850

(56) References cited:
- EP-A1- 1 017 332
- JP-A- 2001 112 743
- JP-A- 2013 192 695
- US-A1- 2011 129 058
- US-A1- 2011 218 426
- US-A1- 2013 157 218
- US-A1- 2015 118 640
- US-A1- 2017 000 430
- US-A1- 2017 000 430
- PECK ET AL: "The variability of condylar point pathways in open-close jaw movements", THE JOURNAL OF PROSTHETIC DENTISTRY, ELSEVIER, AMSTERDAM, NL, vol. 77, no. 4, 1 April 1997 (1997-04-01), pages 394-404, XP005179398, ISSN: 0022-3913, DOI: 10.1016/S0022-3913(97)70165-3

## Description

The invention relates to a method of recording of the temporomandibular joint - TMJ movement defining its geometry and parameters of the stomatognathic system. In particular, the invention enables obtain geometric model of work - anatomical functioning of the TMJ representing the envelope of movement of the condylar head and spatial tracing and mapping of work dynamics of cartilage and ligaments of the TMJ and can be used in diagnostics, surgery, orthodontics and prosthodontics, and especially in orthognathic surgery.

The stomatognathic system is a morphological and functional system of tissues and organs within the oral cavity and facial skeleton that constitutes a functional whole and participates in the process of feed intake, i.e. chewing, oral digestion and swallowing as well as sound articulation, breathing and expressing emotions.

In the partial or full dental reconstruction and the reconstruction of facial skeleton deformities, it is crucial to restore or generate normal function of the stomatognathic system, particularly in a way that the created relations between the TMJ work and the occlusion and the articulation of dental arches provide a normal and stable work of the stomatognathic system. A long-lasting use of the produced dental prosthetic restorations is possible only if the dental prosthetics and precisely adjusted to the parameters of work and geometry of the TMJ.

In the case of orthognathic surgical procedures, it is possible to design the change of the maxillary and mandibular bone base position to reduce gnathic defects and malocclusion. The changes in the position of anatomical parts of the stomatognathic system are designed manually by the surgeon while performing the procedure as the fragments of the maxilla and/or mandible are cut off, which changes their spatial position as well as their pattern of work - functioning - in the stomatognathic system, and, by connecting mandibular bone fragments, the surgeon sets the new position of the condylar head in the TMJ that has to match the work of the stomatognathic system. This method relies on the dexterity and experience of the surgeon. Therefore, a precise knowledge and understanding of individual parameters of TMJ movement and geometry is of utmost importance since the stability of morphological changes introduced to the facial skeleton in the process of surgical correction of gnathic defects and malocclusion can be provided only if the dental restoration is precisely adjusted to the individual parameters of work and geometry of the TMJ. Furthermore, in prosthodontics and maxilla-facial surgery, it is important to obtain the most accurate possible geometric model of TMJ work based on tracing the joint geometry and movement parameters of the stomatognathic system elements so that this model could be used later on in procedures for an individual tracing of each element of the stomatognathic system, including by creating models with the use of printing and milling techniques.

Known methods used in prosthodontics and orthognathic surgery in prosthetic laboratories and prosthodontic clinics involve the articulator that is a device used for the simulation of movements of the mandible in relation to the maxilla, which makes it easier to tailor the restoration of lacking fragments of entire teeth as well as enables carrying out a mandibular movement test while designing surgical correction of the facial skeleton. Articulators make it possible only to adjust dental restorations and mandibular movement tests according to the top-down assumed standards regarding the physiological movements of the stomatognathic system resulting from normal anatomy, including the condylar head in relation to other joint structures involved in speech, chewing, breathing and swallowing. In the case of articulators, it is assumed that the hinge axis of the TMJ, which is the rotary axis of the condyloid process, takes always certain position in relation to other anatomical structures within the skull. These top-down assumed standards very often do not comply with the actual condition. Very frequent abnormalities of the stomatognathic system, particularly dental and gnathic defects, or teeth abrasion lead to the changes in the TMJ mobility and geometry parameters by way of compensation, which results in the TMJ working differently compared to the standards assumed for articulators.

Additionally, modular elements are used in known articulators that imitate anatomical parts of the TMJ, i.e. the condylar head as well as the fossa and the prominence of the joint. They have the shape of a ball that moves in plastic inserts in order to simulate mandibular movements. These elements are standardized and move according to, at maximum, only a few possible patterns of guidance and several different steepness levels - inclination angles of the articular prominence. With the use of the several universal fixed elements, the attempt to trace non-standard individually variable movements of the TMJ and its geometry cannot be precise. Due to both anatomical complexity of this joint and many parameters, especially acquired features that influence the work as well as the geometry of the TMJ, the use of fixed elements is a simplified assumption. Fixed elements do not provide an actual tracing of individual TMJ's work - anatomical functioning and do not derive from the dynamics of this joint.

The anatomy of the TMJ is mainly based on elastic elements: ligaments, muscles and articular disk that determine the dynamics of its work. The set of these elements is hereinafter referred to as the cartilage and ligament apparatus of the TMJ. Taking into consideration the dynamics of soft tissues during the work of the TMJ enables the identification of an optimal position of the condylar head referred to as the central relation. The central relation represents the relation between the mandible and the maxilla, where the condyloid process touches the thinnest part of the articular disc and this refers to the position, in which the condyle is situated in the joint in a comfortable position depending on the dynamics of the soft tissues. Often, it is the position, in which the hinge axis of the TMJ is different than that resulting from the assumed anatomical standards. In the central relation, the condyles are in a rotation position around their own axles without any shift. It is a repetitive position and is considered as a starting point for the preparation of prosthetic restorations (S. Majewski: Gnatofizjologia stomatologiczna, in Polish PZWL 2007). The central relation determines the accuracy of orthodontic, surgical and prosthetic treatment. The incompliance of the treatment with the central relation leads to the development of pathologies and diseases of the TMJ. On the other hand, setting the central relation, which is the most optimal position of the mandible in relation to the maxilla, based on the assumption of certain standards is rather not precise and very superficial.

The well-known methods of dental restoration and those used for the purposes of surgical operations involve also recording the relative position and spatial relations between the position of the maxillary dental arch and the hinge axis of the TMJ with the use of a face-bow and recording the maximum clenching of dental arches using plastic materials in order to then transfer them to articulators.

The method of recording the relation of the mandible to the maxilla consists in transferring the anatomical points of the face to the articulator with the use of a face-bow. The points include: external acoustic pores, frontonasal suture and the nasal bone - the fossa and the infra-orbital point. The use of these points carries a high probability of error resulting from the assumed standards - especially mean values and the top-down assumption regarding the spatial relations between the external auditory meatus and the physiological hinge axis of the TMJ. Therefore, the parameters of TMJ movement and its geometry are arbitrarily determined, particularly the rotary axis of the TMJ, which further translates into the transferring of this geometry to the articulator and eventually results in an imprecise prosthetic diagnosis.

By applying mean values, all the patients whose condition differs from the top-down assumptions and standards are omitted. In patients with all types of malocclusion, developmental abnormalities of jaws, missing teeth and disorders of the TMJs, the probability of the relation between the external auditory canal and the rotary axis of the heads of mandibular condyloid processes meeting the mean value threshold assumed for this method is reduced. This leads to the lack of precise representation of the actual work geometry of the TMJ and obtaining an imprecise model of the joint, which then leads to producing a prosthesis that does not comply with the actual geometry in articulators.

Additionally, the face-bow and the articulator used in prosthodontics and orthognathic surgery are complicated and very time-consuming in use.

The most frequent problem of prosthetic rehabilitation of patients is the lack of a precise adjustment of prosthetic fillings to the factual work - anatomical functioning including geometry and range of movement of the TMJ. Producing prosthetics on a model of stomatognathic system using an articulator results in discrepancies between the shape of prosthetic filling for an individual anatomy of a particular patient and the shape of the filling created based on the scopes of movement in the articulator. Such discrepancies are in each case adjusted in the patient's oral cavity because every change in the shape of dental restoration is immediately signaled by the patient as an obstacle. On the one hand, this procedure requires a greater amount of time, and on the other, it is based on the skills and experience of the technician whose task is to adjust the fillings in the oral cavity. During the adjustment, the doctor can never be sure whether the scope of correction is sufficient and precise. What is more, the correction of ready-made fillings affects the mechanical and aesthetic features of the fillings themselves. Prosthetic restorations that were insufficiently corrected over time become the cause of TMJ disorders, leading to the damage of other components of the stomatognathic system.

There are techniques of maxillary movement reconstruction that are based on the combination of two processes: recording the movement of the maxilla in real time and a static imaging, particularly x-ray tomography.

The use of an intraoral marker/magnetic or electromagnetic marker as a passive element of the system for the imaging of the oral cavity containing the source of X-ray radiation, control system of the imaging system, and means for sending the measurement signals of the relative spatial positions of the marker and the radiation source is disclosed in document WO 2012127117. The system allows setting the x-ray radiation source perpendicularly to the marker and pointing the radiation beam to the center of the marker. The system does not allow recording the movements of anatomical components of the patient's maxilla.

In US20160262711 there is disclosed the maxillary movement tracking system consisting of a kit for maxillary movement detection with at least one camera recording the movement of passive elements such as light reflecting markers, being the points of reference situated on a skeleton construction attached to the patient's forehead and lower dental arch. Moreover, this system is equipped with a skull x-ray imaging device connected to a kit for mandibular movement detection that together form an integral device. The way of tracking the movement of the maxilla using this system consists in obtaining an anatomical image of maxillary hard tissues and locating reference points that coincide with the light reflecting objects situated within the facial skeleton in the obtained anatomical image that are subsequently transferred via visualization system in order to develop a digital model of work of the mandible in relation to the other part of the facial skeleton image, taking the maxillary movement of the patient into consideration. The image processing software generates a virtual model of reference that determines the spatial relation between the reference objects and the anatomical reconstruction recorded as a static image. This solution allows to create volumetric images of jaws with TMJ components, where it is possible to show volumetric images of the mandible in different positions. This solution offers the reconstruction of only a relative movement of volumetric images of the regions of the patient's head between the TMJ components. It does not provide the possibility to obtain a precise geometry and parameters of the TMJ, especially for the purposes of the recreation of the TMJ's work on real models. A disadvantage of the solution is the necessity to equip the examined patient with a set of extra-oral sensors attached with special supporting structures. According to the presented methodology, the method of reference point calibration is divided into several steps, which can cause the accumulation of errors in the positioning of the system as a whole. The size and geometry of the mandibular movement detection kit reduce comfort of the examined patient and make it impossible to apply it in patients with disorders of the maxillofacial apparatus, especially when the upper dental arch overlaps the lower dental arch entirely in a vertical plane. This method cannot be applied if the patient is outside the cranial imaging device.

The method of recording the work of the TMJ with the use of x-ray images analyzed together with video images, on which the common point of reference appears, is disclosed in JP8117214. This solution does not allow to obtain a spatial image of the geometry of the TMJ, especially the space occupied by the articular disc.

The use of mixed sensors that are the combination of gyro and accelerometric sensors to be applied for the configuration of settings of a classic articulator is disclosed in EP 2 363 066.

The method of obtaining a virtual model of work of the teeth, maxilla and mandible based on theoretical assumptions for the relation between the hinge axis of the TMJ and the possible trajectories that may be followed by the mandibular head is disclosed in EP 1017 332. The method consists in transferring the conventionally applied location of the mandibular hinge axis in relation to reference point on 3D models of the maxilla and the mandible using software. One of the possible applications involves the use of mixed sensors that are the combination of gyro and accelerometric sensors, whereas the recorded signals are used in a virtual model to be applied in a classic articulator; the sensors are not used to position the mandibular head in the joint. The method does not allow to obtain a solid figure - geometry - representing the work of the TMJ, especially depicted as a surface or volume and does not provide the possibility to visualize directly or indirectly the components such as the articular disk and ligaments with the capsule.

The article of PECK ET AL: "The variability of condylar point pathways in open-close jaw movements", in THE JOURNAL OF PROSTHETIC DENTISTRY, ELSEVIER (vol. 77, no. 4, 1 April 1997, pages 394-404, XP005179398, ISSN: 0022-3913) discloses a computer method of recording and analysing TMJ movement. It combines Xray and optical images of the jaw each with reference markers. Several points on the condyle are identified on a horizontal cross section of a CT scan, said points are indexed/correlated with the fiducial markers that are use during motion capture of the jaw. The trajectory of said points are obtained for various jaw movements. The trajectory of the individual tracing points are shown in the form of individual curves 4 or 3 points are also presented together in a form of square or a triangle showing the trajectory of each point in relation to each other.

During the invention development, according to the clinical-experimental studies carried out under the guidance of specialists at the Non-Public Health Care Centre for Treatment of Malocclusion, it has been concluded that, thanks to the developed methodology of recording and transferring the images of the stomatognathic system with the use of sensors recording parameter adequate for the defined reference and measurement points such as the values of displacement and/or angular position, it is possible to create a three-dimensional model of the TMJ work reflecting its functioning, especially in the form of a three-dimensional surface tracing the trajectory of work of the condylar head in the TMJ, its geometry and movement trajectory. The very precise and repetitive method of virtual spatial tracing of the dynamics of the cartilage and ligament apparatus in the working TMJ was obtained. The developed method makes it possible to obtain an individual and precise dimensional model of the TMJ with the tracing of the actual geometry of the joint and the dynamic parameters of movement of the stomatognathic system elements, thus also the representation of the actual - anatomically individual parameters of the TMJ - its functioning.

The object of the present invention is defined by the wording of claim 1.

According to the invention, the mandible and skull base movements are recorded by a measurement system comprises sensors to be placed on the patient's head, and particularly accelerometric, gyro, or mixed (gyro-accelerometric) sensors. The sensors record the angular position and/or acceleration generating electronic or electric signals that are send to a data processing unit. Recording the change of angular position and/or the accelerations makes it possible to visualize the change of position of a virtual solid figure in the coordinate system, especially the figure representing the image of mandible with condyloid processes in relation to the skull base.

According to the invention, the method of recording the movement and geometry of the TMJ comprises the step of obtaining a static image of anatomy of the stomatognathic system tissues preferably taken by means of cone beam tomography, computed tomography, computed magnetic resonance image MRI, or ultrasound and obtaining of a spatial image of the geometry of the upper and lower dental arches, especially 3D image, in the form of a solid figure that is presented as a grid of polygons and/or surfaces and/or volumes and/or set of points and/or set of surfaces and/or set of volumes. Then, the obtained static image and the spatial image of the geometry of the upper and lower dental arches are positioned with respect to each other, creating a virtual hybrid model that, by means of a computer programme - an algorithm, is processed into a virtual spatial geometric model of the stomatognathic system in the form of a solid figure presented as a grid of polygons and/or surfaces and/or volumes and/or set of points and/or set of surfaces and/or set of volumes. On the virtual spatial geometric model, the image - geometry of the skull base with the maxilla is selected - separated from the mandibular geometry with the condylar heads. The spatial geometric model serves as a base for creating a virtual, digital, reference XYZ coordinate system by marking at least three reference points in the area of the skull base image - volume as well as for creating a virtual measurement XYZ coordinate system by marking at least three measurement points in the area of the mandible image - volume. Furthermore, an actual - physically realistic measurement system is created with at least one reference sensor for recording the values of displacement and/or angular position within the space of a local reference coordinate system as well as with at least one measuring sensor for recording the values of displacement and/or angular position within the space of a local measurement coordinate system. It is created in the way that physical - anatomical position of at least one reference sensor corresponds to at least one reference point marked on the virtual geometric model and physical position of at least one measurement sensor correspond with at least one measurement point marked on the virtual geometric model. Then, during the movement of the mandible, both in occlusion and during articulation movements, signals are being recorded by measurement and reference sensors and are being transferred into the virtual spatial geometric model by recording the movement of the mandible volume in a virtual measurement coordinate system in relation to the skull base volume. Subsequently, the recorded movements of the mandible volume are being added up for creating the image of the spatial movement of the mandible volume. In the obtained image of the spatial movement of the mandible volume, tracing points are selected to image - map the condylar head by setting subsequent positions in time for the tracing points based on the recorded movement of the mandible volume. Then, the trajectory of individual tracing points is created in the form of curves between which the surfaces extend. This step lead to formation a dimensional surface - 3D - that traces - maps the work of the condylar head in the TMJ - the individual functioning of the condylar head based on shown in the image - the dimensional surface movement range, the joint mobility, and its geometry.

Preferably, a dynamic tracing - mapping of a three-dimensional volume of the cartilage and ligament apparatus is carried out by marking a three-dimensional surface of the TMJ fossa on the spatial virtual model of the stomatognathic system and then connecting the obtained surface of the TMJ fossa surface with the obtained three-dimensional surface mapping the condylar head work - functioning. Then, a surface is created between the external outlines of both obtained surfaces that closes the space between them.

Preferably, the signals from the measurement and reference sensors are transferred onto the virtual spatial geometric model by marking the changes of position of the measurement sensor or the changes of position of the local measurement coordinate system that are transferred onto a global coordinate system on the virtual spatial geometric model of the stomatognathic system, and by marking the changes of position of the reference sensor or the changes of position of the local reference coordinate system that are transferred onto the global coordinate system and the differences corresponding to the relative change of positions of the reference and measurement points are subsequently marked - determined. Then, in the global coordinate system on the virtual spatial geometric model of the stomatognathic system, the changes of position of the local reference coordinate system are deducted
- subtracted from the changes of position of the local measurement coordinate system.

Preferably, the virtual spatial geometric model is created in the form of a spatial volume presented as a grid of polygons, preferably triangles, that is divided into an independent volume of the skull base and an independent volume of the mandible.

Preferably, the obtained images in the form of a solid figure are saved in STL format.

Preferably, during the method, the spatial image of the geometry of the upper and lower dental arches is obtained by scanning the upper and lower dental arches.

Preferably, gyro and/or accelerometric and/or gyro-accelerometric sensors are used as measurement and/or reference sensors.

Preferably, the orientation - positioning of the virtual reference coordinate system coincides with, corresponds to the local reference coordinate system and the orientation of the virtual measurement coordinate system correspond to the local measurement coordinate system.

The invention refers to the development of a spatial geometric model of the stomatognathic system with a precise image of the parameters especially joints mobility and geometry of the TMJ work - functioning based on an individual anatomy and physiology of the TMJ. The spatial geometric model has the form of a solid figure shown as a grid of polygons, particularly triangles and/or volumes and/or surfaces and/or set of points and/or set of volumes and/or set of volumes. The invention is applicable in orthognathic procedures and surgeries in patients with the TMJ work significantly diverging from the assumed standards and assumptions, which frequently occurs as a result of malocclusion or gnathic defects. The method can be carried out - applied when the patient is outside the skull imaging device.

According to the invention, the method is particularly suitable for the development of a dimensional surface - 3D image - mapping, tracing the trajectory of condylar head work in the TMJ in its extreme positions, enabling an indirect reconstruction of the geometry and physiology of individual anatomical elements of the TMJ that participate in the movement such as the articular disc, capsule, and lateral ligaments.

Thanks to this effect, the invention makes it possible to create a spatial geometric model of the TMJ that includes the TMJ-surrounding soft tissues, in further description referred to as the virtual tracing of the cartilage and ligament apparatus of the TMJ. The so-far known methods has been providing the possibility to obtain the image of the mandible and the skull base exclusively.

The invention enables the production of prosthetic restorations in compliance with the actual individual anatomical characteristics and scopes of TMJ movement. It offers the possibility to record the position of the condylar head in central relation based on the observation of work of the elements of the stomatognathic system.

Furthermore, the invention enables the creation of a geometric model - a solid figure - of the TMJ, based on which the surgeon sees in real time how the condylar head is positioned in the TMJ. This solution combined with prosthetic restorations enables a dramatic increase in the accuracy of the performed orthognathic procedures and the stability of their results, i.e. in the work of the reconstructed stomatognathic system.

According to the invention, the method makes it also possible to reproduce the actual movement trajectory of the joint on a physical real model. Thanks to the invention, there is also a possibility to produce anatomical elements of the TMJ in the form of plastic models that can be applied as the components of an individual anatomic articulator, which then serve as a medium to transfer individual spatial relations between the components of the stomatognathic system precisely onto a real object. Thanks to the transfer of spatial relations between the actual trajectory of work of the condylar head and the shape of dental arches onto a model, by means of which prosthetic restorations are adjusted, dental technician prepares the prosthetic restoration that is fully tailored to the individual work of the stomatognathic system - occlusion and articulation - already at the laboratory, which reduces the time dedicated for the adjustment of the prosthesis at the dentist's office as well as contributes to the durability of the restoration and its correct functioning. The invention makes it possible to create a virtual tracing of the cartilage and ligament apparatus of the TMJ in the full range of movement of the mandible in relation to the skull base, which is individual for every patient. Based on the produced virtual elements, it is possible to create real components that can be used especially for the construction of an individual anatomic articulator that would take into consideration the actual and not the simplified movement. An individual anatomic articulator is an articulator that enables the use of components produced in the form of virtual models of the upper and lower dental arches and the condylar heads as well as a three-dimensional surface tracing the trajectories of condylar head work for the purposes of recreating them by means of printing or milling techniques in such a way that the spatial relations between the components are identical with the virtual geometric model of the stomatognathic system.

A more detailed description of the embodiment of the invention is provided in the examples and in the figures. Figure 1 shows a diagram of the system for recording the movement of the TMJ. Figure 2 presents a diagram of the procedure of creating a three-dimensional surface that traces the dynamics of work of the condylar head. In Figure 3, the process of carrying out a three-dimensional imaging is depicted. Figure 4 illustrates the process of taking impressions of dental arches with the use of modelling paste. In Figure 5, the process of scanning the dental arch geometry with an intraoral scanner is shown. Figure 6 shows a volume or surface in the form of a triangular grid that was generated in the scanning process. Figure 7 illustrates the process of positioning of the triangular grid representing the geometry of dental arches in relation to the static anatomical image. In Figure 8, a hybrid model can be seen that is a combination of the triangular grid with a voxel cloud in spatial relation to the dental arches. Figure 9 presents the distribution of points of reference in a static anatomical image, whereas Figure 10 shows the distribution of points of reference on the dental arch scans. Figure 11 depicts a virtual spatial model of the stomatognathic system. Figure 12 shows the distribution of reference and measurement points on a virtual spatial model and a diagram illustrating the construction of virtual coordinate systems, whereas in Figure 13, the distribution of reference and measurement points with a diagram illustrating the construction of local coordinate systems is shown. Figure 14 presents the record of subsequent positions of the mandible volume during abduction. Figure 15 shows the sum of the mandible volume in time while building a new volume both in transverse and sagittal plane view, i.e. a shape of three-dimensional surface tracing the trajectory of movement of the condylar head in longitudinal and transverse sections. In Figure 16, the method of marking points within the condylar head region can be observed.

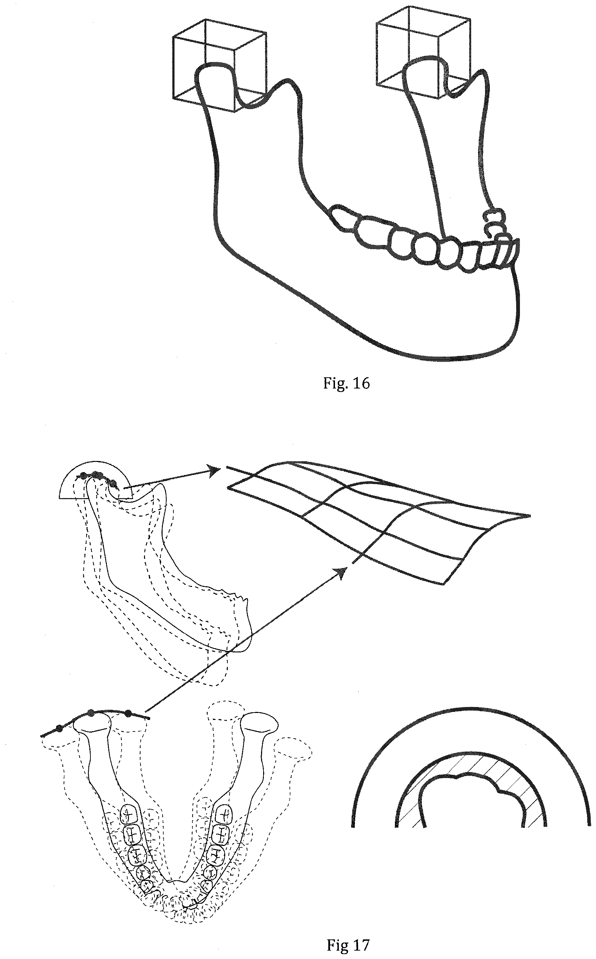

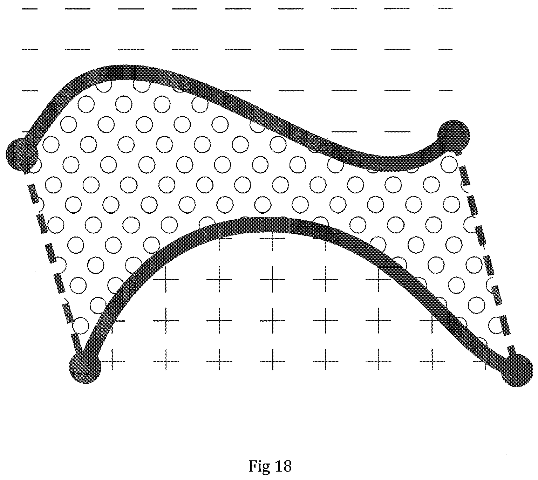

Figure 17 presents the method of creating a three-dimensional surface tracing the trajectories of movement of the condylar head. Figure 18 shows the method of creating the volume tracing the dynamic work of the condylar head by creating a closing surface.

### Example 1

### 1. Creating the TMJ movement and geometry recording system.

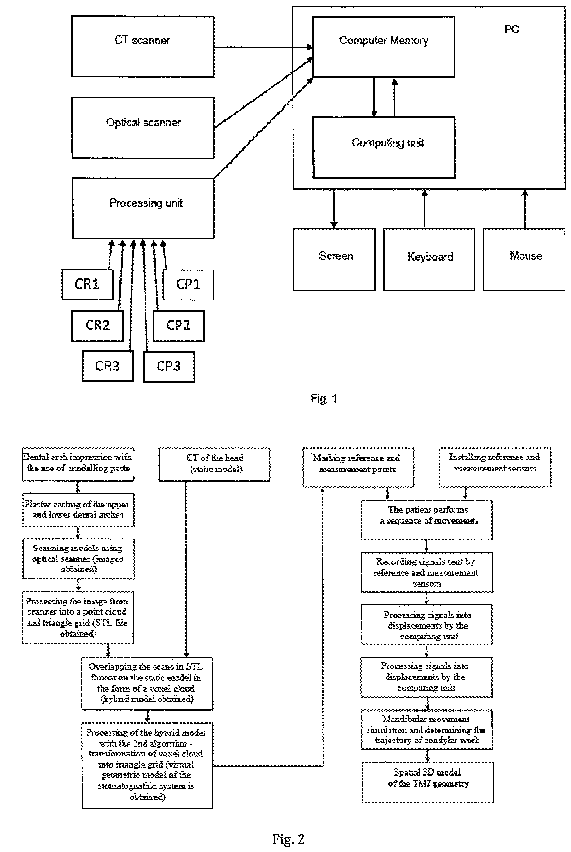

The TMJ movement recording system is shown in Figure 1. The measurement system for movement recording works with the CT scanner, optical scanner and contains six accelerometric sensors as well as a processing unit, a computing unit and a computer memory. The computing unit is a part of the computer connected to a monitor, keyboard and mouse. The processing unit sends signals to the computer memory in which all data is stored. The computing unit processes the signals, information and data saved in the computer memory.

In the computing unit, the first algorithm is implemented whose task is to process the image from the optical scanner into a point cloud, and then into a triangular grid.

In the computing unit, the second algorithm is implemented whose task is to position the scans of the upper and lower dental arches in relation to the static image of the stomatognathic system.

In the computing unit, the third algorithm is implemented whose task is to process the voxel cloud into a triangular grid.

### 2. Method of recording the movement and geometry of the TMJ.

All stages of this method are shown in Figure 2.
a) Obtaining a static anatomical image.

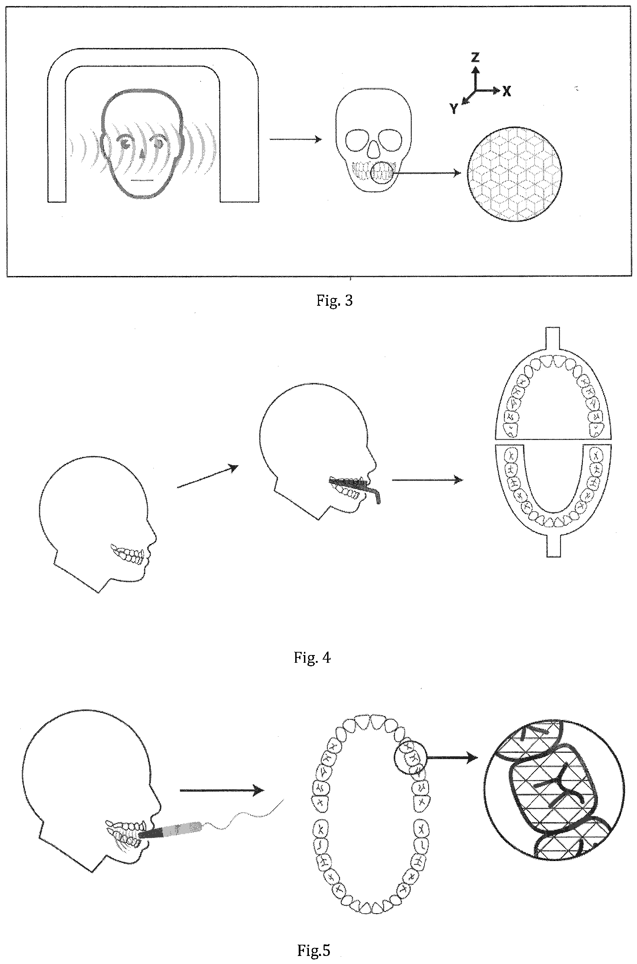

As indicated in Figure 3, cone beam computer tomography (CBCT) of the skull is carried out via transmission imaging where the images, being individual tissue layers of the head region, are saved in DICOM (Digital Imaging and Communications in Medicine) format.

As a result, a static anatomical image of the stomatognathic system tissues is obtained. The image is stored in the memory of the computing unit. It is also possible to obtain a static anatomical image using other imaging techniques, including ultrasound imaging with the use of algorithms in order to transform it into a three-dimensional image.
b) Obtaining a spatial image of the dental arch geometry.

As indicated in Figure 4, impressions of the upper and lower dental arches are taken using modelling paste for plaster casting. They are scanned with an optical scanner and saved in the memory of the computing unit.

Another possibility to obtain the image of the dental arches is to scan them directly using an intraoral scanner, as shown in Figure 5.

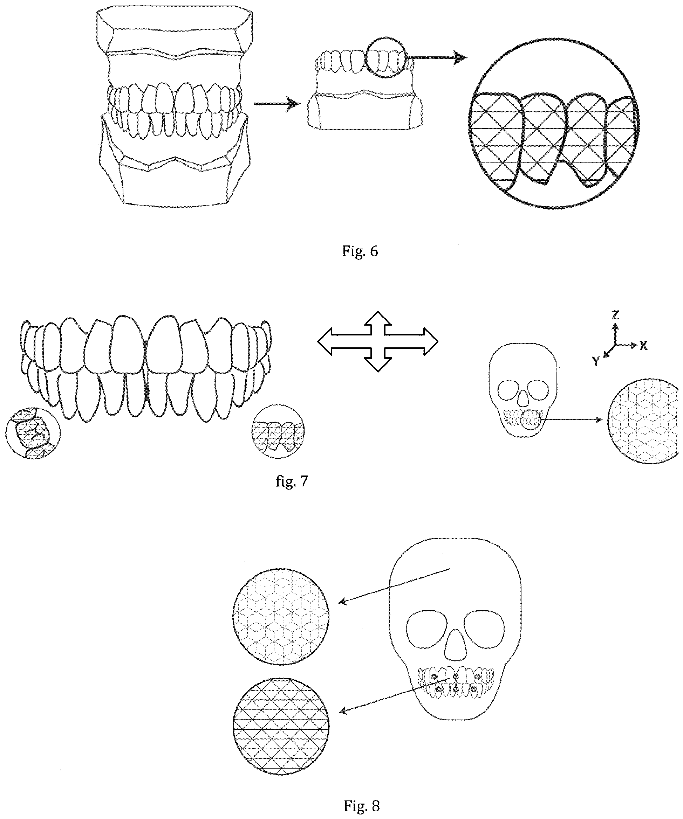

The obtained scan result comes in the form of an image that is processed with the help of the first algorithm in the computing unit into a point cloud and then into a triangular grid and saved as data in STL (stereolithography) format in the computing unit memory. As a result, at this stage, a spatial image of the surfaces of the lower and upper dental arches is obtained, i.e. of the external and upper side that is a region whose size is limited by curves or the volume of the lower and upper dental arches i.e. a space enclosed by surfaces, as shown in Figure 6. The obtained surfaces or volumes are further referred to as the scan of the upper and lower dental arches. The spatial image of the geometry of the dental arches can be saved also in a different image format as a solid figure shown in the form of a polygon grid, particularly triangle grid, volume, or surface, or the set of points, the set of surfaces, or the set of volumes.
c) Positioning the upper and lower arch scan in relation to the static image of the stomatognathic system and creating a hybrid model containing the stomatognathic system image.

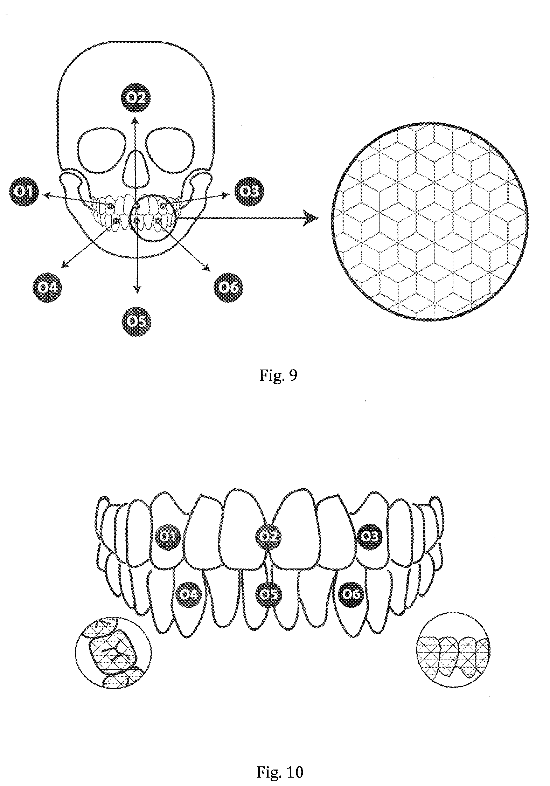

The CBCT static image of the stomatognathic system is positioned in relation to the upper and lower dental arch scan that has the form of a triangle grid, as shown in Figure 7, and they are linked according to the same anatomical position. Consequently, a virtual hybrid model is created as depicted in Figure 8. The hybrid model consists partially of a voxel cloud and partially of a triangle grid. Automatic positioning is carried out in the computing unit where the static image of the stomatognathic system moves with the use of the second algorithm in relation to the upper and lower dental arch scan in the form of a triangle grid. With the use of the algorithm, the position being the minimum difference of volume between the static image and the upper and lower dental arch scan is determined. Another positioning option is to apply reference points 01, 02, and 03 on the scan on the upper dental arch and reference points 04, 05, and 06 on the lower dental arch in the places where the anatomical positions coincide both to the static image, as shown in Figure 9, and to the upper and lower dental arch scan, as depicted in Figure 10. Then, the reference points marked on the upper and lower dental arch scan are positioned so that the reference points 01, 02, 03, 04, 05, and 06, referring to each anatomical position, comply with corresponding reference points in the static image. The result is saved in the computing unit memory.
d) Constructing a virtual spatial geometric model of the stomatognathic system.

The virtual hybrid model is transformed into a virtual spatial geometric model of the stomatognathic system using the third algorithm in the computing unit.

The virtual spatial geometric model of the stomatognathic system is a spatial volume presented in the form of a triangle grid - the skull base volume, containing anatomical elements such as the toothed maxilla connected to other bone elements of the skull base visible on imaging with the articular fossa of the TMJ and an independent mandible volume comprising such anatomical elements as the toothed mandible with the two condylar heads. All the anatomical elements of the virtual geometric model are placed according to a common global XYZ coordinate system.

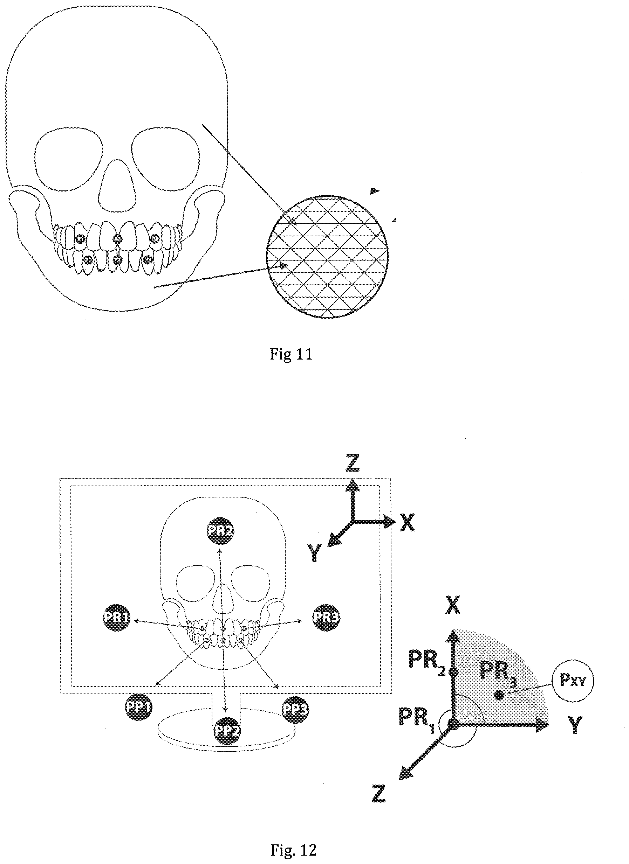

The virtual spatial geometric model is constructed in the following way: a curve is determined on each layer on one part of the hybrid model that consists of the voxel cloud obtained using CSCT based on the grey-scale analysis, being the border between the hard tissue and soft tissue with its surroundings. As a result, a set of curves is obtained that are situated in layers on parallel planes with a distance between each layer corresponding to the distance between each picture that together create an image of the tissue in DICOM format that is a voxel cloud. Then, surfaces are extended between the curves of adjacent layers, creating a set of surfaces between each layer. The curves of the extreme layer form flat surfaces limited by these curves. After that, the adjacent surfaces are connected with each other and their ends are connected with the flat surfaces, forming a closed volume. This. volume is filled with a triangle grid. As a result, a virtual spatial geometric model of the stomatognathic system is built, tracing independently the mandible volume and the skull base volume, as shown in Figure 11.

The obtained virtual spatial geometric model is saved in STL format in the computer's memory.

The obtained geometric model of the stomatognathic system can be also stored in other format that allows saving spatial images - solid figures, presented as polygon grids, especially triangle grids, volumes or surfaces, or their sets. Preferably, the image is created in the form of a volume.

On the virtual spatial model, the trajectory of the condylar head in the TMJ is traced and the reference and measurement points are marked, to which the displacement and rotation values are transferred after being measured with the use of the respective reference and measurement sensors.
e) Positioning of sensors in relation to the points on virtual spatial geometric model and their orientation in space.

On the virtual spatial geometric model of the stomatognathic system that differentiates independently between the mandible volume and the skull base volume, three reference points PR1-3 are marked on the skull base and three measurement points PP1-3 are marked on the mandible, as shown in Figure 12.

The reference points marked on the virtual spatial geometric model within the skull base region, i.e. in the obtained volume of the skull base, create a virtual reference coordinate system WLUW1 with the first reference point PR1 representing the zero intercept, the x-axis is represented by a straight line that crosses the first reference point PR1 and the second reference point PR2, and the XY plane of the virtual local coordinate system WLUW1 extends onto the positions of all reference points PR1, PR2, and PR3. The y-axis of the virtual reference coordinate system WLUW1 is determined by the 90-degree rotation of the x-axis in the XY plane in relation to the zero intercept of the virtual reference coordinate system WLUW1. The z-axis is represented by an axis that is perpendicular to the x and y axles.

The measurement points marked on the virtual spatial geometric model of the mandible volume build a virtual measurement coordinate system WLUW2 in the area of the mandible volume of the virtual spatial geometric model with the first measurement point PP1 representing the zero intercept, the x-axis is represented by the straight line that crosses the first measurement point PP1 and the second measurement point PP2, and the XY plane of the virtual measurement coordinate system WLUW2 extends onto the positions of all measurement points PP1, PP2, and PP3. The y-axis of the virtual measurement coordinate system WLUW2 is determined by a 90-degree rotation of the x-axis in the XY plane in relation to the zero intercept of the virtual measurement coordinate system WLUW2. The z-axis is represented by an axis that is perpendicular to the x and y axles.

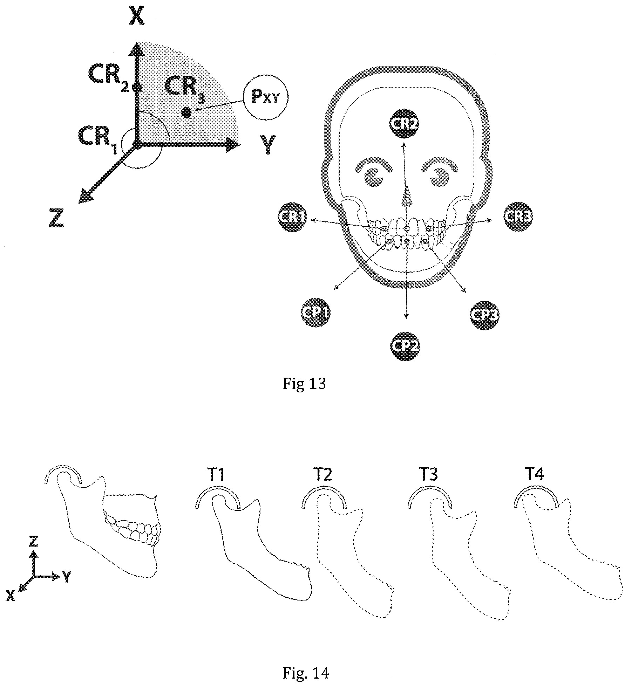

As shown in Figure 13, three accelerometric reference sensors CR are placed in the region of patient's skull base, i.e. on the anatomical surfaces of the upper dental arch teeth, whose anatomical position coincides with, correspond to the reference points marked on the virtual geometric model, and three accelerometric measurement sensors CP on the patient's mandible, i.e. on the anatomical surfaces of the lower dental arch teeth, whose anatomical position coincides with, correspond to the measurement points marked on the virtual geometric model.

The reference sensors CR attached to the skull base form a local reference coordinate system LUW1 with the first reference sensor CR1 representing the zero intercept, the x-axis is represented by a straight line that crosses the first reference sensor CR1 and the second reference sensor CR2, and the XY plane of the local coordinate system LUW1 extends onto the positions of all reference sensors CR1, CR2, and CR3. The y-axis of the coordinate system LUW1 is determined by the 90-degree rotation of the x-axis in the XY plane in relation to the zero intercept of the local reference coordinate system LUW1. The z-axis is represented by an axis that is perpendicular to the x and y axles.

The LUW1 and WLUW1 coordinate systems overlap. The position of the local reference coordinate system LUW1 coincides with, reflects the same anatomic orientation as the virtual reference coordinate system WLUW1.

The measurement sensors CP attached to the mandible form the local measurement coordinate system LUW2 with the first measurement sensor CP1 representing the zero intercept, the x-axis is represented by a straight line that crosses the first measurement sensor CP1 and the second measurement sensor CP2, and the XY plane of the local coordinate system LUW2 extends onto the positions of all measurement sensors CP1, CP2, and CP3. The y-axis of the coordinate system LUW2 is determined by the 90-degree rotation of the x-axis in the XY plane in relation to the zero intercept of the local measurement coordinate system LUW2. The z-axis is represented by an axis that is perpendicular to the x and y axles. There is a similar coincidence between the LUW2 and WLUW2 coordinate systems.

It is possible to determine differently the local reference and measurement coordinate systems and the virtual reference and measurement coordinate systems; however, the positions of reference sensors coincide with the reference points marked on the virtual spatial geometric model and the positions of measurement sensors coincide with the measurement points marked on a virtual geometric model, i.e. the positions of the respective local coordinate systems in relation to the anatomy in material reality and the positions of the respective virtual coordinate system in virtual reality are the same. The positions of reference sensors correspond to the reference points marked on the virtual spatial geometric model and the positions of measurement sensors correspond to the measurement points marked on a virtual geometric model.
f) Recording virtual spatial movement of the mandible volume.

In the following step, the processing unit records signals form the measurement and reference sensors. The initial position of the mandible in relation to the skull base is assumed to be the same on a virtual geometric model of the stomatognathic system as the initial position of the mandible in relation to the skull base of the patient during the measurement.

After defining the initial position, the patient performs a sequence of movements in occlusion as well as articulation movements reaching as far as the maximum positions in all planes, i.e. mandibular movements in occlusion, during which the teeth of the opposing arches stay in contact and then, one by one, anterior mandibular movements, lateral movements to the left and to the right, posterior movement as well as articulation movements, during which the teeth of the opposing arches do not touch each other with their surfaces, and then, subsequently, the abduction and adduction movements of the mandible followed by the lateral mandibular movements to the left and to the right. The recorded subsequent positions of the mandible volume during abduction - T1, T2, T3, and T4 on the virtual spatial model are shown in Figure 14.

During mandibular movements, signals sent by the measurement sensors CP1-3 and reference sensors CR1-3 are recorded. These signals contain information on the changes of acceleration values in three directions. The signals are sent by the processing unit to the memory of the computing unit. Then, the computing unit processes them mathematically, i.e. performs a double integration in order to obtain displacement values of the reference sensors CR1-3 and measurement sensors CP1-3 in three directions.

The results are the values of displacement of the first reference sensor CR1 in three directions, representing the displacement of the zero intercept of the LUW1 local coordinate system in three directions. Based on the difference of displacement results for the first reference sensor CR1 and the second reference sensor CR2, the rotation angle of the skull base in relation to the y and z axles is calculated, and at the same time, also the rotation angle of the local reference coordinate system LUW1. Based on the difference of displacement results for the reference sensors CR1 and CR3 as well as CR1 and CR2, the rotation angle in relation to the x-axis is calculated.

The results in the form of values of displacement of the first measurement sensor CP1 in three directions is represented by the displacement of the zero intercept of the LUW2 coordinate system in three directions. Based on the difference of displacement results for the first measurement sensor CP1 and the second measurement sensor CP2, the rotation angle of the mandible in relation to the y and z axles is calculated. Based on the difference of displacement results for the measurement sensors CP1 and CP3 as well as CP1 and CP2, the rotation angle in relation to the x-axis of the LUW2 is calculated.

Then, in a global coordinate system on a virtual spatial geometric model of the stomatognathic system, the computing unit deducts the displacements and rotations of the local reference coordinate system LUW1 from the displacements and rotations of the local measurement coordinate system LUW2. This calculation is performed as follows: the displacement of the global coordinate system that crosses the zero intercept of the LUW1 in the x direction is deducted from the displacement of the global coordinate system that crosses the zero intercept of the LUW2 in the x direction, the displacement of the global coordinate system that crosses the zero intercept of the LUW1 in the y direction is deducted from the displacement of the global coordinate system that crosses the zero intercept of the LUW2 in the y direction, the displacement of the global coordinate system that crosses the zero intercept of the LUW1 in the z direction is deducted from the displacement of the global coordinate system that crosses the zero intercept of the LUW2 in the z direction, the rotation angle in relation to the x-axis of the global coordinate system that crosses the zero intercept of the LUW1 is deducted from the rotation angle in relation to the axis parallel to the x-axis of the global coordinate system that crosses the zero intercept of the LUW2, the rotation angle in relation to the y-axis of the global coordinate system that crosses the zero intercept of the LUW1 is deducted from the rotation angle in relation to the axis parallel to the y-axis of the global coordinate system that crosses the zero intercept of the LUW2, the rotation angle in relation to the z-axis of the global coordinate system that crosses the zero intercept of the LUW1 is deducted from the rotation angle in relation to the axis parallel to the z-axis of the global coordinate system that crosses the zero intercept of the LUW2, obtaining as a result the mandibular movement in relation to the skull base.

Then, after having considered the skull base movement, the obtained displacements and rotations of the mandible volume in relation to the skull are an excitation, i.e. they are imposed on the mandible volume movement in relation to the stationary skull base volume in the virtual geometric model of the stomatognathic system in the computing unit by imposing displacements in the directions parallel to the x, y, and z axles of the global coordinate system as well as rotations of the virtual measurement coordinate system WLUW2 in relation to the axis parallel to the x, y, and z axles of the global coordinate system that crosses the zero intercept of the WLUW2. Therefore, it is decided that here, the virtual reference coordinate system WLUW1 is stationary. This method makes it possible to obtain the movement of the mandible volume in the virtual measurement coordinate system WLUW2 and in the global coordinate system in relation to the stationary volume of the skull base. Another method to achieve this result is to assume that the observation and measurement system for the mandibular volume movement is located on the skull base volume. Consequently, regardless of the movements of the skull base volume, the mandibular volume movements are observed and measured in relation to the skull base.

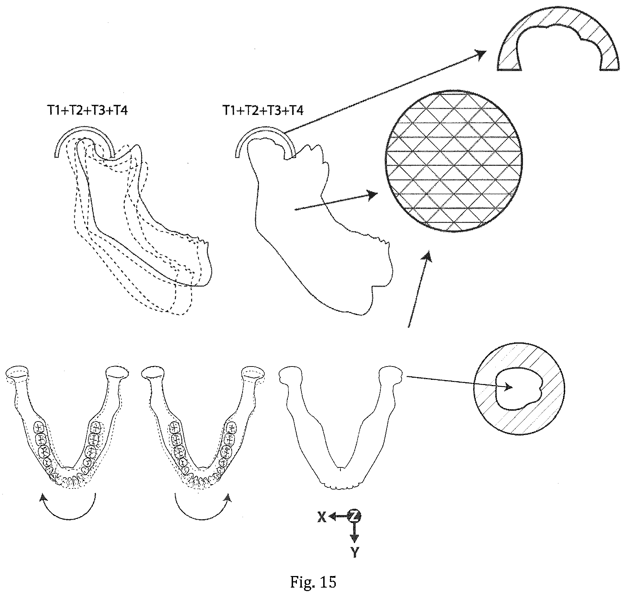

As a result, the complete virtual movement of the mandible volume is obtained, being the sum of the recorded movements T1-T4, as shown in Figure 15. The movement of the mandible volume is presented from a lateral perspective and in a top-down view.
g) Determining the virtual geometry of a three-dimensional surface tracing the trajectory of the condylar work in the TMJ - the TMJ movement trajectory.

As shown in Figure 16, all the points of the triangle grid that trace the condylar head are selected in the computing unit from the set of points of the triangle grid that form a virtual spatial geometric model in the area of the recorded mandibular volume movement. These points build a geometric volume of the condylar head, whose movement trajectory is determined within the TMJ. The changes of position of the mandible during the mandibular volume movement T1-T4 are changing the position of the condylar head, thus they change the position of the triangle grid points tracing the condylar head. The changes in point position are recorded on the virtual spatial model of the stomatognathic system as changes of coordinates in the global coordinate system for each selected point. Then, for each selected point, a movement trajectory is created by drawing segments or curves that connect with each other the nearest positions held by each point in the global coordinate system. Based on the segments or curves, a surface is drawn that includes the subsequent positions of each individual point during the movement of the mandible volume, as shown in Figure 17. As a result, a set of surfaces for all selected points is obtained, determining an empty space on the virtual geometric model, in which the condylar head volume moves and which is represented by a three-dimensional surface tracing the trajectory of condylar work in the TMJ.

It is possible to continue to create the image in the form of a solid figure representing the cartilage and ligament apparatus as a volume that is a dynamic representation of the three-dimensional volume of the cartilage and ligament apparatus, where all the volume elements are positioned in relation to each other, enabling the creation of real models of the stomatognathic system in the form of individual anatomic articulators. Therefore, it is possible to make prosthetic restorations based on models that enable tracking the each patient's individualized TMJ movements, thereby allowing the patient who uses the restorations to use them immediately without the necessity to make any shape corrections and providing a correct work of the TMJ without the risk of incidence of any TMJ disorders.

The volume of the cartilage and ligament apparatus is created by determining a three-dimensional surface of the TMJ articular fossa from the skull base volume on the virtual spatial model of the stomatognathic system. Then, the obtained surface of the articular fossa is linked to the obtained three-dimensional surface tracing the condylar work in the computing unit. The next step is to create a surface between the external outlines, closing the space between these surfaces, i.e. around the two surfaces - the surface that traces the trajectory of the condylar work in the TMJ and the surface tracing the TMJ articular fossa on the skull base on the virtual spatial model of the stomatognathic system. The closing surface is created by extending a new surface between the curve of the external outline of one surface and the curve of the external outline of the other surface, as shown in Figure 18.

The three-dimensional surface tracing the trajectory of condylar work in the TMJ and the surface of the TMJ articular fossa on the skull base on the virtual spatial model of the stomatognathic system as well as the closing surface build together a closed volume that fills the space between these surfaces entirely.

In this way, a volume is obtained that contains the elements of the TMJ such as the articular disc, the capsule and the ligaments of the TMJ. This enables an indirect visualization of the articular disc geometry during the work of the joint, which provides a virtual tracing of the cartilage and ligament apparatus of the TMJ.

The obtained surfaces build a closed surface that traces virtually the cartilage and ligament apparatus of the TMJ within the entire range of the mandibular movement in relation to the skull base, which is individual for each patient. Based on the obtained virtual elements, it is possible to produce real elements with great precision that can be used while constructing an individual anatomic articulator, taking into consideration the actual, and not simplified, movement.

The images created in the whole sequence from the point a to g - volumes or surfaces representing the skull, the mandible and the virtual volume tracing the cartilage and ligament apparatus are displayed on the monitor screen. All solid figures - surfaces and volumes are editable in terms of generating stomatognathic system models with the use of 3D printing, thus in terms of reproducing the model of the stomatognathic system in a prosthetic laboratory.

The presented methodology makes it possible to:
- record parameters of mandibular movement in relation to the skull base as well as the movements of the mandible and the skull base in relation to the global coordinate system, which provides the accuracy of orthognathic surgical procedures,
- determine a dimensional surface, 3D image tracing and mapping the trajectory of condylar head volume in the TMJ - the surface that limits from below the structures such as the articular disc, which makes it possible to reproduce - obtain the actual - real and individual parameters of the trajectory of TMJ movement on accurate and precise model, including e.g. by reconstructing the geometry of joint components with the use of modular elements,
- create a virtual tracing - mapping of the cartilage and ligament apparatus of the TMJ in the form of an image as a volume, i.e. a space limited by virtual surfaces that represents - reflects also image of soft tissues within the TMJ, in which the movement of the TMJ is being traced. The volume is defined as a space limited by: a three-dimensional surface mapping the trajectory of condylar head work in the TMJ, the surface of the articular fossa of the TMJ on the skull base marked on the obtained virtual spatial geometric model, and the surface that closes the two other surfaces.

The method allows also:
- to record such a position of the condylar head that corresponds to its central relation, which enables a correct and precise provision of orthodontic, surgical and prosthetic treatment,
- to build a model, in which spatial relations of the elements of the stomatognathic system shall trace - reflect the real model of the patient.

### Example 2

### 1. Creating the TMJ movement recording system.

The system is designed as described in Example 1 except that instead of three accelerometric reference sensors CR1, CR2, and CR3 attached to the skull base, 1 accelerometric reference sensor CR1 is used (measuring the acceleration in all 3 directions) and 2 gyro reference sensors CR2 and CR3 (measuring the angular position in all 3 directions each) and, instead of 3 accelerometric measurement sensors CP1, CP2, and CP3 attached to the mandible, 1 accelerometric measurement sensor CP1 is used (measuring the acceleration in all 3 directions) and 2 gyro measurement sensors CP2 and CP3 (measuring the angular position in all 3 directions each).

### 2. Method of recording the TMJ movement.

The TMJ movement is recorded as described in Example 1 except that:
- instead of cone beam tomography, MRI is used to obtain the static model;
- instead of scanning the impression of the upper and lower dental arches taken with the use of modelling paste, the dental arches are scanned directly using an intraoral scanner;
- instead of placing three accelerometric reference sensors on the skull base, one accelerometric reference sensor CR1 and two gyro reference sensors CR2 and CR3 are placed;
- instead of placing three accelerometric measurement sensors on the mandible, one accelerometric measurement sensor CP1 and two gyro measurement sensors CP2 and CP3 are placed;
- results in the form of the acceleration value recorded by the accelerometric reference sensor CR1 are integrated twice afterwards in order to obtain displacement values; the results in the form of displacement values from the first sensor CR1, that represents the zero intercept of the local coordinate system of the skull base LUW1, represent the displacement in three directions of the zero intercept of the local coordinate system LUW1; the results in the form of the changes in angular position values from the gyro reference sensors CR2 and CR3 are transferred to the local coordinate system LUW1, which makes it possible to rotate the local coordinate system LUW1.
- results in the form of the acceleration value recorded by the accelerometric measurement sensor CP1 are integrated twice afterwards in order to obtain displacement values; the results in the form of displacement values from the first sensor CP1, that represents the zero intercept of the local coordinate system of the mandible LUW2, represent the displacement in three directions of the zero intercept of the local coordinate system LUW2; the results in the form of the changes in angular position values from the gyro measurement sensors CP2 and CP3 are transferred to the local coordinate system LUW2, which makes it possible to rotate the local coordinate system LUW2.

### Example 3

### 1. Creating the TMJ movement recording system.

The system is designed as described in Example 1 except that instead of three reference accelerometric sensors CR1, CR2, and CR3 attached to the skull base, 1 mixed (gyro-accelerometric) reference sensor CR1 is used (measuring both the acceleration and the angular position in all 3 directions) and, instead of 3 measurement accelerometric sensors CP1, CP2, and CP3 attached to the mandible, 1 mixed (gyro-accelerometric) measurement sensor CP1 is used (measuring the acceleration and the angular position in all 3 directions).

### 2. Method of recording the TMJ movement.

The TMJ movement is recorded as described in Example 1 except that instead of cone beam tomography, CT scan is performed to obtain the static model and instead of using an optical scanner to scan the impression of the upper and lower dental arches taken with the use of modelling paste, the dental arches are scanned with the use of an intraoral scanner.

Instead of placing three accelerometric reference sensors on the skull base, one mixed gyro-accelerometric reference sensor CR1 is placed, whereas, instead of placing three accelerometric measurement sensors on the mandible, one mixed gyro-accelerometric measurement sensor CP1 is placed.

The results in form of the acceleration value recorded by the accelerometric reference sensor CR1 are integrated twice afterwards in order to obtain displacement values. The results in the form of displacement and rotation values from the sensor CR1, that represents the zero intercept of the local coordinate system of the skull base LUW1, represent the displacement of the zero intercept of the local coordinate system LUW1 in three directions and the rotation in relation to the three axles of to the local coordinate system LUW1.

The results in the form of the acceleration value recorded by the accelerometric measurement sensor CP1 are integrated twice afterwards in order to obtain displacement values. The results in the form of displacement and rotation values from the first sensor CP1, that represents the zero intercept of the local coordinate system of the mandible LUW2, represent the displacement of the zero intercept of the local coordinate system LUW2 in three directions and the rotations in relation to the three axles of to the local coordinate system LUW2.

In this way, the local coordinate system LUW1 is determined by the position of the mixed reference sensor which coincides with the zero intercept of the coordinate system. The x-axis is determined by creating an axis that crosses through the reference sensor and the point at the apex of the mesiobuccal cusp of tooth #27. The XY plane of the local coordinate system LUW1 is created by extending it through the three points: the position of the reference sensor, the point at the apex of the mesiobuccal cusp of tooth #27, and the point at the apex of the mesiobuccal cusp of tooth #17. The z-axis is represented by an axis that is perpendicular to the XY plane and crosses the zero intercept of the LUW1 coordinate system. The y-axis is created by rotating the x-axis 90° in relation to the z-axis.

The local coordinate system LUW2 is determined by the position of the mixed measurement sensor on the mandible which coincides with the zero intercept of the coordinate system. The x-axis is determined by creating an axis that crosses the measurement sensor and the point on the tooth #37. The XY plane of the local coordinate system LUW2 is created by extending it through the three points: the position of the measurement sensor, the point on the tooth #37, and the point on the tooth #47. The z-axis is represented by an axis that is perpendicular to the XY plane and crosses the zero intercept of the LUW2 coordinate system. The y-axis is created by rotating the x-axis 90° in relation to the z-axis.

The local reference coordinate system WLUW1 in the region of the skull base volume of the virtual geometric model is determined by the position of the mixed reference sensor which coincides with the zero intercept of the coordinate system. The x-axis is determined by creating an axis that crosses the reference point and the point on the tooth #27. The XY plane of the local coordinate system WLUW1 is created by extending it through the three points: the position of the reference point, the point on the tooth #27, and the point on the tooth #17. The z-axis is represented by an axis that is perpendicular to the XY plane and crosses the zero intercept of the WLUW1 coordinate system. The y-axis is created by rotating the x-axis 90° in relation to the z-axis.

The local measurement coordinate system WLUW2 in the region of the mandibular volume of the geometric model is determined by the position of the measurement point which coincides with the zero intercept of the coordinate system. The x-axis is determined by creating an axis that crosses the measurement point and the point on the tooth #37. The XY plane of the local coordinate system WLUW2 is created by extending it through the three points: the position of the measurement point, the point on the tooth #37, and the point on the tooth #47. The z-axis is represented by an axis that is perpendicular to the XY plane and crosses the zero intercept of the WLUW2 coordinate system. The y-axis is created by rotating the x-axis 90° in relation to the z-axis.

## Claims

1. Method of recording the movement and geometry of the temporomandibular joint TMJ that comprises step of obtaining a static image of the head anatomy and step of tracing the mandibular movement wherein, a static image of tissues of
the stomatognathic system is taken, preferably by means of cone beam tomography or computer tomography CT or magnetic resonance imaging MRI or ultrasound imaging, and a spatial image of the geometry of the upper and lower dental arches is obtained, wherein the image is obtained in the form of a solid figure presented as a polygon grid and/or surfaces and/or volumes and/or a set of points and/or a set of surfaces and/or a set of volumes, and then, the static image as well as the spatial image of the geometry of the upper and lower dental arches are positioned in relation to each other, creating a virtual hybrid model that is transformed using an algorithm into a virtual spatial geometric model of the stomatognathic system being represented by a solid figure in the form of a polygon grid and/or surfaces and/or volumes and/or a set of points and/or a set of surfaces and/or a set of volumes, wherein the geometry of the skull base with the maxilla is selected on the virtual spatial geometric model independently from the geometry of the mandible with condylar heads, whereas, on the spatial geometric model, a virtual reference coordinate system XYZ (WLUW1) is built by marking at least three reference points within the skull base image and a virtual measurement coordinate system XYZ (WLUW2) is built by marking at least three measurement points within the mandible image, and furthermore, a physical measurement system is built that contains at least one reference sensor that records the values of displacement and/or angular position within the space of the local reference coordinate system (LUW1) and whose anatomical position correspond to at least one reference point of the position marked on the virtual geometric model as well as at least one measurement sensor that records the values of displacement and/or angular position within the space of the local measurement coordinate system (LUW2) and whose anatomical position corresponds to at least one measurement point marked on the virtual geometric model and then, during mandibular movements, both in occlusion and during articulation movements, signals from measurement and reference sensors are being recorded and transferred into the virtual spatial geometric model by recording the movement of the mandible volume in the virtual measurement coordinate system (WLUW2) in relation to the skull base volume and then the recorded movements of the mandible volume are being added to create the image of the spatial mandibular volume movement and then, on the obtained image of the spatial mandibular volume movement, tracing points for mapping the condylar head are selected, for which subsequent positions in time are being determined based on the recorded movement of the mandible volume, and then the trajectory of the individual tracing points in the form of curves are being created, between which surfaces are extended, creating a dimensional surface that maps the condylar head functioning within the TMJ.

2. Method according to the claim 1, wherein a dynamic mapping of the dimensional volume of the cartilage and ligament apparatus is performed by marking a dimensional surface of the articular fossa of the TMJ on the virtual spatial model of the stomatognathic system and then the obtained surface of the articular fossa is connected to the obtained dimensional surface mapping the functioning of the condylar head, and a closing surface between external outlines of the obtained surfaces is created.

3. Method according to the claim 1 or 2, wherein the signals from the measurement and reference sensors are transferred onto the virtual spatial geometric model by determining the position changes of the measurement sensors or the position changes of the local measurement coordinate system (LUW2) that are transferred onto a global coordinate system on the virtual spatial model of the stomatognathic system and by determining the position changes of the reference sensors or the position changes of the local reference coordinate system (LUW1) that are transferred onto the global coordinate system and then the differences corresponding to relative change of position of reference points and measurement points are determined and then in the global coordinate system on the virtual spatial geometric model of the stomatognathic system the position changes of the local reference coordinate system (LUW1) are deducted from the position changes of the local measurement coordinate system (LUW2)

4. Method according to any of the claim 1-3, wherein the virtual spatial geometric model is created in the form of a spatial volume presented using a polygon grid, preferably triangle grid, that is divided into an independent skull base volume and an independent mandible volume.

5. Method according to any of the claims 1-4, wherein the images obtained in the form of a solid figure are saved in STL format.

6. Method according to the claim 1, wherein the spatial image of the geometry of the upper and lower dental arches is obtained by scanning the upper and lower dental arches.

7. Method according to the claim 1, wherein gyro sensor and/or accelerometric sensor and/or gyro-accelerometric sensor are used as measurement and/or reference sensors.

8. Method according to the claim 1, wherein the method is performed in the way that the orientation of the virtual reference coordinate system (WLUW1) correspond to the local reference coordinate system (LUW1), and the orientation of the virtual measurement coordinate system (WLUW2) correspond to the local measurement coordinate system (LUW2).

## Patentansprüche

1. Verfahren zum Aufzeichnen einer Kiefergelenkbewegung und -geometrie, das einen Schritt umfasst, bei dem ein statisches Bildes der Kopfanatomie gemacht wird und einen weiteren Schritt, bei dem die Bewegung des Unterkiefers verfolgt wird, wobei ein statisches Bild von Geweben des stomatognathen Systems aufgenommen wird, vorzugsweise mithilfe der Kegelstrahl-Computertomographie oder Computertomographie CT oder Magnetresonanztomographie MRT oder der Ultraschall-Bildgebung, und ein räumliches Bild der Geometrie der oberen und unteren Zahnbögen erstellt wird, wobei das Bild in Form eines räumlichen Körpers generiert wird, die als Polygongitter und/oder Flächen und/oder Räume und/oder eine Anzahl von Punkten und/oder eine Anzahl von Flächen und/oder eine Anzahl von Räumen dargestellt wird, und dann das statische Bild sowie das räumliche Bild der Geometrie des oberen und unteren Zahnbogens zueinander in Verbindung gesetzt werden, wodurch ein virtuelles Hybridmodell entsteht, das mit Hilfe eines Algorithmus in ein virtuelles räumliches geometrisches Modell des stomatognathen Systems umgewandelt wird, das durch einen räumlichen Körper in Form eines Polygongitters und/oder von Flächen und/oder Räumen und/oder einer Anzahl von Punkten und/oder einer Anzahl von Flächen und/oder einer Anzahl von Räumen dargestellt wird, wobei die Geometrie der Schädelbasis mit dem Oberkiefer auf dem virtuellen räumlichen Geometriemodell unabhängig von der Geometrie des Unterkiefers mit den Kondylarköpfen ausgewählt wird, während auf dem räumlichen Geometriemodell ein virtuelles Referenzkoordinatensystem XYZ (WLUW1) durch Markierung von mindestens drei Referenzpunkten innerhalb der Abbildung der Schädelbasis und ein virtuelles Messkoordinatensystem XYZ (WLUW2) durch Markierung von mindestens drei Messpunkten innerhalb der Abbildung des Unterkiefers aufgebaut werden, ferner wird auch ein physikalisches Messsystem aufgebaut wird, das mindestens einen Referenzsensor umfasst, der die Werte der Verschiebung und/oder der Winkelposition im Raum des lokalen Referenzkoordinatensystems (LUW1) erfasst und dessen anatomische Position mindestens einem Referenzpunkt der auf dem virtuellen geometrischen Modell markierten Position entspricht, sowie mindestens einen Messsensor, der die Werte der Verschiebung und/oder der Winkelposition im Raum des lokalen Messkoordinatensystems (LUW2) erfasst und dessen anatomische Position mindestens einem auf dem virtuellen geometrischen Modell markierten Messpunkt entspricht und dann, während der Bewegung des Unterkiefers, sowohl in Okklusion als auch während der Artikulation, Signale von Mess- und Referenzsensoren aufgezeichnet und in das virtuelle räumliche geometrische Modell übertragen werden, indem die räumliche Bewegung des Unterkiefers im virtuellen Messkoordinatensystem (WLUW2) in Bezug auf die räumliche Darstellung der Schädelbasis aufgezeichnet wird, wonach die aufgezeichneten Bewegungen des Unterkiefers addiert werden, um die Abbildung der räumlichen Unterkieferbewegung zu erhalten, und dann, auf der erhaltenen Abbildung der räumlichen Unterkieferbewegung, werden Abtastpunkte für die Kartierung des Kondylarkopfes ausgewählt, für welche nachfolgende Positionen in der gleichen Zeit auf der Grundlage der aufgezeichneten Bewegung des Unterkiefers bestimmt werden, und dann wird der Verlauf der einzelnen Abtastpunkte in Form von Kurven dargestellt, zwischen denen sich Flächen erstrecken, so dass eine dimensionale Fläche entsteht, die das Funktionieren des Kondylarkopfes im Kiefergelenk abbildet.

2. Verfahren nach Anspruch 1, wobei dem eine dynamische Abbildung des dimensionalen Raums des Knorpel- und Bandapparates erstellt wird, indem eine dimensionale Fläche der Gelenkfossa des Kiefergelenks auf dem virtuellen räumlichen Modell des stomatognathen Systems markiert wird und dann die erhaltene Fläche der Gelenkfossa mit der erhaltenen dimensionalen Fläche, die das Funktionieren des Kondylarkopfes abbildet, in Verbindung gebracht, und eine Schließfläche zwischen den äußeren Umrissen der erhaltenen Oberflächen geschaffen wird.

3. Verfahren nach Anspruch 1 oder 2, wobei die Signale von den Mess- und Referenzsensoren auf ein virtuelles räumliches Geometriemodell übertragen werden, indem die Änderungen der Positionen von Messsensoren oder die Änderungen der Positionen des lokalen Koordinatensystems (LUW2) ermittelt und auf globales Koordinatensystem auf dem virtuellen räumlichen Modell des stomatognathen Systems übertragen werden und indem die Änderungen der Positionen der Referenzsensoren oder die Änderungen der Positionen des lokalen Koordinatensystems (LUW1) bestimmt und auf das globale Koordinatensystem übertragen und dann die Unterschiede in Bezug auf die relative Änderung der Position von Referenz- und Messpunkten ermittelt werden und danach im globalen Koordinatensystem auf dem virtuellen, räumlichen, geometrischen Modell des stomatognathen Systems werden die Änderungen der Position des lokalen Referenzkoordinatensystems (LUW1) von den Änderungen der Position des lokalen Messkoordinatensystems (LUW2) abgezogen.

4. Verfahren nach einem der Ansprüche 1 bis 3, wobei das virtuelle räumliche geometrische Modell in Form eines räumlichen Volumens erstellt wird, das unter Verwendung eines Polygonrasters, vorzugsweise eines Dreiecksrasters, dargestellt wird und in ein unabhängiges Schädelbasisvolumen und ein unabhängiges Unterkiefervolumen unterteilt ist.

5. Verfahren nach einem der Ansprüche 1 bis 4, wobei die in Form eines räumlichen Körpers erhaltenen Bilder im STL-Format gespeichert werden.

6. Verfahren nach Anspruch 1, wobei die räumliche geometrische Abbildung der oberen und unteren Zahnbögen durch Scannen der oberen und unteren Zahnbögen erstellt wird.

7. Verfahren nach Anspruch 1, wobei als Mess- und/oder Referenzsensoren Kreiselsensoren und/oder Beschleunigungssensoren und/oder Kreiselbeschleunigungssensoren verwendet werden.

8. Verfahren nach Anspruch 1, wobei das Verfahren so durchgeführt wird, dass die Orientierung des virtuellen Referenzkoordinatensystems (WLUW1) dem lokalen Referenzkoordinatensystem (LUW1) und die Orientierung des virtuellen Messkoordinatensystems (WLUW2) dem lokalen Messkoordinatensystem (LUW2) entspricht.

## Revendications

1. Procédé d'enregistrement du mouvement et de la géométrie de l'articulation temporo-mandibulaire ATM qui comprend une étape d'obtention d'une image statique de l'anatomie de la tête et une étape de traçage du mouvement mandibulaire, dans lequel une image statique des tissus du système stomatognathique étant prise, de préférence au moyen d'une tomographie à faisceau conique ou d'une tomographie par ordinateur ou d'une imagerie par résonance magnétique IRM ou d'une imagerie par ultrasons, et une image spatiale de la géométrie des arcades dentaires supérieure et inférieure est obtenue, l'image étant obtenue sous la forme d'un solide présenté sous forme d'un quadrillage polygonal et/ou de surfaces et/ou de volumes et/ou d'un ensemble de points et/ou d'un ensemble de surfaces et/ou d'un ensemble de volumes, puis, l'image statique ainsi que l'image spatiale de la géométrie des arcades dentaires supérieure et inférieure sont positionnées l'une par rapport à l'autre, créant un modèle hybride virtuel qui est transformé à l'aide d'un algorithme en un modèle de géométrie spatiale virtuelle du système stomatognathique représenté par un solide en forme de quadrillage polygonal et/ou de surfaces et/ou de volumes et/ou d'un ensemble de points et/ou d'un ensemble de surfaces et/ou d'un ensemble de volumes, la géométrie de la base du crâne avec le maxillaire étant sélectionnée sur le modèle de géométrie spatiale virtuelle indépendamment de la géométrie de la mandibule avec des têtes condyliennes, tandis que, sur le modèle de géométrie spatiale, un système de coordonnées de référence virtuel XYZ (WLUW1) est construit par marquage au moins trois points de référence dans l'image de base du crâne et un système de coordonnées de mesure virtuel XYZ (WLUW2) est construit par marquage au moins trois points de mesure dans l'image de la mandibule, et en outre, un système de mesure physique est construit qui contient au moins un capteur de référence qui enregistre les valeurs de déplacement et/ou de position angulaire dans l'espace du système de coordonnées de référence local (LUW1) et dont la position anatomique correspond à au moins un point de référence de la position marqué sur le modèle de géométrie virtuelle ainsi qu'au moins un capteur de mesure qui enregistre les valeurs de déplacement et/ou de position angulaire dans l'espace du système de coordonnées de mesure local (LUW2) et dont la position anatomique correspond à au moins un point de mesure marqué sur le modèle de géométrie virtuelle puis, lors des mouvements mandibulaires, à la fois dans l'occlusion et pendant les mouvements d'articulation, les signaux des capteurs de mesure et de référence sont enregistrés et transférés dans le modèle de géométrie spatiale virtuelle en enregistrant le mouvement du volume de la mandibule dans le système de coordonnées de mesure virtuel (WLUW2) par rapport au volume de base du crâne et puis les mouvements enregistrés du volume mandibulaire sont additionnés pour créer l'image du mouvement spatial du volume mandibulaire puis, sur l'image obtenue du mouvement spatial du volume mandibulaire, des points de traçage pour cartographier la tête condylienne sont sélectionnés, pour lesquels des positions subséquentes dans le temps sont déterminés sur la base du mouvement enregistré du volume de la mandibule, puis la trajectoire des points de traçage individuels sous la forme de courbes est créée, entre lesquelles des surfaces sont étendues, créant une surface dimensionnelle qui cartographie la tête condylienne fonctionnant dans le ATM.

2. Procédé selon la revendication 1, dans lequel une cartographie dynamique du volume dimensionnel de l'appareil cartilagineux et ligamentaire étant réalisée en marquant une surface dimensionnelle de la fosse articulaire de l'ATM sur le modèle spatial virtuel du système stomatognathique puis la surface obtenue de la fosse articulaire est connectée à la surface dimensionnelle obtenue cartographiant le fonctionnement de la tête condylienne, et une surface de fermeture entre les contours externes des surfaces obtenues est créée.

3. Procédé selon la revendication 1 ou 2, dans lequel les signaux des capteurs de mesure et de référence étant transférés sur le modèle de géométrie spatiale virtuelle en déterminant les changements de position des capteurs de mesure ou les changements de position du système de coordonnées de mesure local (LUW2) qui sont transféré sur un système de coordonnées global sur le modèle spatial virtuel du système stomatognathique et en déterminant les changements de position des capteurs de référence ou les changements de position du système de coordonnées de référence local (LUW1) qui sont transférés sur le système de coordonnées global, puis les différences correspondant au changement relatif de position des points de référence et des points de mesure sont déterminés, puis dans le système de coordonnées global sur le modèle de géométrie spatiale virtuelle du système stomatognathique, les changements de position du système de coordonnées de référence local (LUW1) sont déduits des changements de position du système de coordonnées de mesure local (LUW2)

4. Procédé selon l'une quelconque des revendications 1 à 3, dans lequel le modèle de géométrie spatiale virtuelle étant créé sous la forme d'un volume spatial présenté en utilisant une grille polygonale, de préférence une grille triangulaire, qui est divisée en un volume de base de skuli indépendant et un volume de mandibule indépendant.

5. Procédé selon l'une quelconque des revendications 1 à 4, dans lequel les images obtenues sous la forme d'un solide étant enregistrées au format STL.

6. Procédé selon la revendication 1, dans lequel l'image spatiale de la géométrie des arcades dentaires supérieure et inférieure étant obtenue par balayage des arcades dentaires supérieure et inférieure.

7. Procédé selon la revendication 1, dans lequel un capteur gyroscopique et/ou un capteur accélérométrique et/ou un capteur gyro-accélérométrique étant utilisés comme capteurs de mesure et/ou de référence.

8. Procédé selon la revendication 1, dans lequel le procédé est exécuté de telle sorte que l'orientation du système de coordonnées de référence virtuel (WLUW1) correspondant au système de coordonnées de référence local (LUW1), et l'orientation du système de coordonnées de mesure virtuel (WLUW2) correspondant au système de coordonnées de mesure local (LUW2).
